(19) Europäisches Patentamt
European Patent Office
Office européen des brevets

(11) Veröffentlichungsnummer: **0 061 418**
**B1**

(12) # EUROPÄISCHE PATENTSCHRIFT

(45) Veröffentlichungstag der Patentschrift: **21.08.85**

(51) Int. Cl.⁴: **C 07 J 53/00**, A 61 K 31/585, A 61 K 31/565

(21) Anmeldenummer: **82730038.5**

(22) Anmeldetag: **19.03.82**

(54) **7-Alpha-Alkoxycarbonyl-15-beta.16-beta-methylen-4-androstene, Verfahren zu ihrer Herstellung und Verwendung als Arzneimittel.**

(30) Priorität: **23.03.81 DE 3111951**

(43) Veröffentlichungstag der Anmeldung: **29.09.82 Patentblatt 82/39**

(45) Bekanntmachung des Hinweises auf die Patenterteilung: **21.08.85 Patentblatt 85/34**

(84) Benannte Vertragsstaaten: **AT BE CH DE FR GB IT LI LU NL SE**

(56) Entgegenhaltungen:
**EP - A - 0 019 690**
**FR - A - 2 150 852**
**FR - A - 2 370 755**
**US - A - 4 069 219**

**JOURNAL OF MEDICINAL CHEMISTRY, Band 20, Nr. 10, Oktober 1977, Seiten 1304-1310, Washington D.C., USA, R.M. WEIER et al.: "Synthesis and antimineralocorticoid activities of some 6-substituted 7alpha-carboalkoxy steroidal spirolactones"**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Nickisch, Klaus, Dr., Alt-Lichtenrade 11, D-1000 Berlin 49 (DE)**
Erfinder: **Laurent, Henry, Dr., Glambecker Weg 21, D-1000 Berlin 28 (DE)**
Erfinder: **Wiechert, Rudolf, Prof., Dr., Petzower Strasse 8a, D-1000 Berlin 39 (DE)**
Erfinder: **Casals-Stenzel, Jorge, Dr., Wichernstrasse 20, D-1000 Berlin 31 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 061 418 B1

**Beschreibung**

Die Erfindung betrifft neue 7α-Alkoxycarbonyl-15β,16β-methylen-4-androstenverbindungen der allgemeinen Formel I

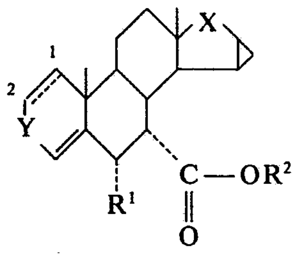

(I)

worin

$R^1$ ein Wasserstoffatom oder eine Methylgruppe,
$R^2$ eine Alkylgruppe mit 1 bis 4 Kohlenstoffatomen,
X die Gruppierung

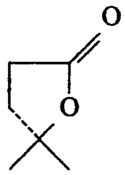

oder

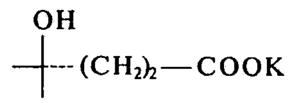

und
Y eine Carbonylgruppe

bedeuten und die Bindung zwischen den Kohlenstoffatomen 1 und 2 gesättigt ist oder eine Doppelbindung darstellt, oder

X die Gruppierung

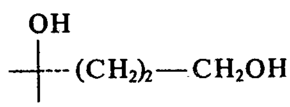

und
Y die Gruppe

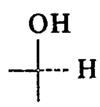

bedeuten, wenn die Bindung zwischen den Kohlenstoffatomen 1 und 2 eine Einfachbindung darstellt.

Die erfindungsgemäßen Verbindungen besitzen wertvolle pharmakologische Eigenschaften. Sie sind u. a. Diuretika vom Typ der Aldosteron-Antagonisten, d. h., sie kehren die Wirkung von Desoxycorticosteron auf die Natrium- und Kaliumausscheidung um. Die erfindungsgemäßen Verbindungen erweisen sich im Testmodell von Hollmann (G. Hollmann et al., Tubuläre Wirkungen und renale Elimination von Spirolactonen, Naunyn-Schmiedebergs Arch. Exp. Path. Pharmak. 247 (1964) 419; P. Marx, Renale Wirkungen des d-Aldosterons und seines Antagonisten Spironolactons, Diss. Med. Fak. FU Berlin 1966) dem bekannten Spironolacton in ihrer Wirkung überlegen.

Es sind bereits zahlreiche andere Steroide mit antimineralkortikoider Wirkung bekannt. In der US-A-069 219 bzw. in der Publikation J. Med. Chem. 20 (1977) 1304-08 werden z. B. 6-substituierte 7α-Carbalkoxysteroid-spirolactone mit dieser Wirkung beschrieben. Desweiteren sind aus der FR-A-2 370 755 strukturanaloge Steroid-spirolactone und deren offenkettige Form, nämlich die 21-Carbonsäuren, bekannt, die sich zu der vorgenannten Verbindung im wesentlichen durch das Fehlen eines Substituenten in 6-Stellung unterscheiden.

Darüberhinaus sind aus der FR-A-2 370 755 solche strukturanalogen Steroid-spirolactone bekannt, die in der 7α-Stellung eine —SCOR-Gruppe aufweisen.

Die erfindungsgemäßen Verbindungen mit einer Substituenten-Kombination der —OCOR-Gruppe in 7α-Stellung und einer Methylengruppe in 15β,16β-Stellung erwiesen sich überraschenderweise diesen bekannten Verbindungen in ihrer Hauptwirkung überlegen.

Die Erfindung betrifft ferner ein Verfahren zur Herstellung von 7α-Alkoxy carbonyl-15β,16β-methylen-4-androstenverbindungen der allgemeinen Formel I:

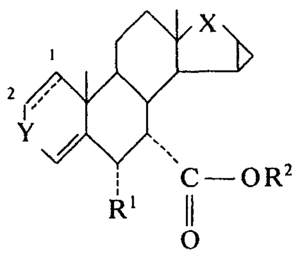

(I)

worin

$R^1$ ein Wasserstoffatom oder eine Methylgruppe,
$R^2$ eine Alkylgruppe mit 1 bis 4 Kohlenstoffatomen,
X die Gruppierung

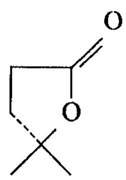 oder 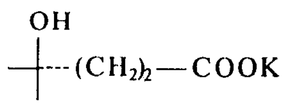

und
Y eine Carbonylgruppe

bedeuten und die Bindung zwischen den Kohlenstoffatomen 1 und 2 gesättigt ist oder eine Doppelbindung darstellt, oder

X die Gruppierung

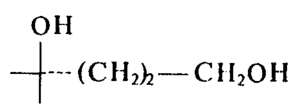

und
Y die Gruppe

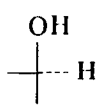

bedeuten, wenn die Bindung zwischen den Kohlenstoffatomen 1 und 2 eine Einfachbindung darstellt, welches dadurch gekennzeichnet ist, daß man in an sich bekannter Weise eine Verbindung der allgemeinen Formel II

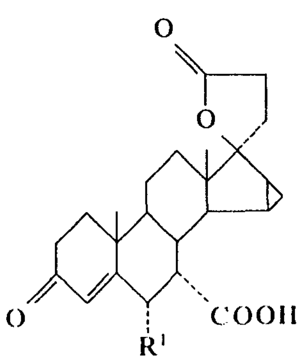

(II)

worin $R^1$ die obengenannte Bedeutung besitzt, zu einem nieder-Alkyl-Ester umsetzt und gegebenenfalls die $\Delta^1$-Doppelbindung einführt und/oder den Lacton-Ring zum entsprechenden Propionsäuren-Kalium-Salz öffnet oder zu den entsprechenden 3β,17β-Dihydroxy-15β,16β-methylen-17α-(3-hydroxypropyl)-4-androsten-7α-alkoxycarbonylverbindungen reduziert.

Die Veresterung der 7α-Carboxylverbindungen der Formel II erfolgt nach an sich bekannten Methoden, indem man die Carbonsäure mit Diazoalkanen, beispielsweise mit Diazomethan oder Diazoäthan, in einem geeigneten Lösungsmittel wie Diethyläther, Tetrahydrofuran oder Dioxan oder einem Gemisch dieser Lösungsmittel bei einer Temperatur von 0° bis 25°C, vorzugsweise bei 0 bis 5° umsetzt, anschließend überschüssiges Diazoalkan durch Zusatz einer organischen Säure wie Eisessig oder Weinsäure zersetzt und die Lösung im Vakuum vom Lösungsmittel befreit. Die Veresterung kann auch durchgeführt werden, indem man nach den dem Fachmann bekannten Methoden die Carbonsäure der Formel II mit Chlorameisensäure-alkylester, wie beispielsweise Chlorameisensäurebutyl- oder isobutyl-Ester, in einem geeigneten Lösungsmittel, wie Tetrahydrofuran oder Dioxan, in Gegenwart eines tertiären Amins, wie beispielsweise Triethylamin als HCl-Fänger, bei einer Temperatur von 0°C bis 20°C, vorzugsweise bei 0°C bis 5°C zum gemischten Anhydrid der allgemeinen Formel III umsetzt

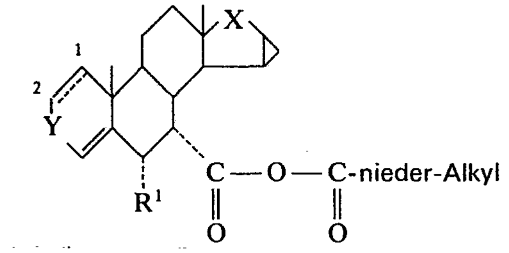

(III)

in der $R^1$, X und Y die obengenannte Bedeutung haben.

Zur Aufarbeitung wird der gebildete Niederschlag des tertiären Amin-Hydrochlorids abfiltriert und das Filtrat vom Lösungsmittel befreit, wobei man das gemischte Anhydrid der Formel III als Rohprodukt erhält.

Um den gewünschten Ester der allgemeinen Formel II zu erhalten, löst man die Verbindung der Formel III in einem Alkohol $R^2-OH$ (mit $R^2$ in der oben angegebenen Bedeutung), erhitzt 24 bis 72 Stunden, vorzugsweise 48 Stunden, zum Sieden, destilliert danach das Lösungsmittel ab und isoliert die Verbindung der Formel II.

Die Einführung der $\Delta_1$-Doppelbindung erfolgt nach an sich bekannten Methoden und kann auf chemischem oder mikrobiologischem Wege erfolgen. Geeignete chemische Dehydrierungsmittel zur 1,2-Dehydrierung sind beispielsweise Selendioxid, 2,3-Dichlor-5,6-dicyanobenzochinon, Chloranil, Thalliumtriacetat oder Bleitetraacetat.

Geeigente Mikroorganismen für die 1,2-Dehydrierung sind beispielsweise Schizomyceten, insbesondere solche der Genera Arthrobacter, wie z. B. simplex ATCC 6946; Bacillus, wie z. B. B. lentus ATCC 13805 und B. sphaericus ATCC 7055; Pseudomonas, wie z. B. P. aeruginosa IFO 3505; Flavobacterium, wie z. B. F. flavescens IFO 3058; Lactobacillus, wie z. B. L. brevis IFO 3345 und Nocardia, wie z. B. N. opaca ATCC 4276.

Die 1,2-Dehydrierung wird bevorzugt chemisch ausgeführt. Hierzu wird das 1,2-Dihydrosteroid in einem geeigneten Lösungsmittel mit dem Dehydrierungsmittel über längere Zeit erwärmt. Geeignete Lösungsmittel sind beispielsweise Dioxan, tert. Butanol, Tetrahydrofuran, Toluol, Benzol bzw. Gemische dieser Lösungsmittel.

Die Reaktion ist nach mehreren Stunden abgeschlossen. Es empfiehlt sich, die Umsetzung durch Dünnschichtchromatographie zu verfolgen. Das Reaktionsgemisch wird aufgearbeitet, wenn das Ausgangsmaterial umgesetzt ist.

Die sich gegebenenfalls anschließende Öffnung des Lactonringes erfolgt ebenfalls nach an sich bekannten Methoden. Hierzu wird das Lacton mit verdünnter Kalilauge erwärmt, wobei sich das Kaliumsalz der 3-substituierten Propionsäure bildet.

Nach beendeter Reaktion wird das Reaktionsgemisch in üblicher Weise, z. B. durch Fällung, Extraktion, Umkristallisation und/oder Säulenchromatographie, aufgearbeitet.

Ist im Verlaufe des erfindungsgemäßen Verfahrens die Reduktion der 3-Carbonylgruppe sowie des Lacton-Ringes zu den erfindungsgemäßen Verbindungen der Formel I, worin $R^1$ und $R^2$ die obengenannte Bedeutung besitzen, jedoch

X die Gruppierung

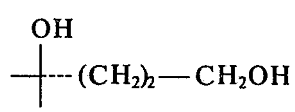

und

Y die Gruppe

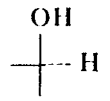

darstellen und die Bindung zwischen den Kohlenstoffatomen 1 und 2 eine Einfachbindung ist, erforderlich, so wird diese Reduktion bevorzugt stufenweise durchgeführt, indem man die 3-Keto-spirolactone der Formel I, worin $R^1$ und $R^2$ die obengenannte Bedeutung besitzen und

X die Gruppierung

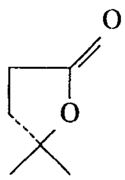

Y eine Carbonylgruppe

und die Bindung zwischen den Kohlenstoffatomen 1 und 2 eine Einfachbindung darstellt, in einem geeigneten Lösungsmittel, wie beispielsweise wasserfreiem Diethyläther oder Tetrahydrofuran, löst und bei −50 bis −70°C, vorzugsweise bei −70°C, mit einem Reduktionsmittel wie beispielsweise Diisobutylaluminumhydrid, gelöst in Toluol, umsetzt und die Lösung nach beendeter Reduktion, deren Ende durch Dünnschichtchromatographie kontrolliert wird, mit einer organischen Säure, wie gesättigter Zitronensäurelösung, zersetzt. Nach Isolierung des Reduktionsproduktes durch Ausschütteln mit einem mit Wasser nicht mischbaren Lösungsmittel, wie Methylenchlorid oder Chloroform, Neutralwaschen dieser Lösung mit Wasser, Trocknen und Eindampfen, erhält man die Verbindung der allgemeinen Formel IV

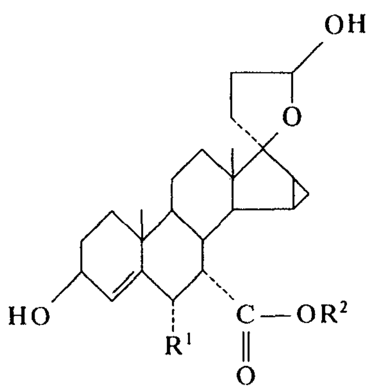

(IV)

mit $R^1$ und $R^2$ in der obengenannten Bedeutung, als Rohrprodukt.

Es wird in einem geeigneten Lösungsmittel, wie beispielsweise i-Propanol gelöst, die Lösung mit einem Reduktionsmittel, wie beispielsweise Natriumborhydrid oder Lithiumaluminium-tri-tert.-butoxyhydrid in absolutem Tetrahydrofuran mehrere Stunden zum Sieden erhitzt, nach beendeter Umsetzung mit verdünnter Mineralsäure, wie verdünnter Schwefelsäure oder Phosphorsäure, versetzt und das Reduktionsprodukt mit einem mit Wasser nicht mischbaren Lösungsmittel extrahiert.

## Herstellung der Ausgangsverbindungen

A) 3-(7α-Carboxy-17β-hydroxy-15β,16β-methylen-3-oxo-4-androsten-17α-yl)-propionsäurelacton:

1. 5,3 g 3-(17β-Hydroxy-15β,16β-methylen-3-oxo-4,6-androsta-dien-17α-yl)-propionsäurelacton (Herstellung siehe DE-OS 2 652 761) werden in 100 ml absolutem Tetrahydrofuran gelöst und unter Argon mit 30 ml einer 2molaren Lösung von Diethylaluminiumcyanid in Toluol versetzt, 30 Minuten am Rückfluß gekocht, abgekühlt und in 100 ml 1n Natronlauge gegeben. Nach Extraktion mit Dichlormethan wäscht man mit 20%iger Schwefelsäure und Wasser, trocknet über Natriumsulfat und engt im Vakuum ein. Durch Chromatographie des Rohproduktes an Kieselgel mit Hexan/Aceton erhält man 5 g 3-(7α-Cyano-17β-Hydroxy-15β,16β-methylen-3-oxo-4-androsten-17α-yl)-propionsäurelacton vom Schmelzpunkt 238—241°C.
$\alpha_D = 68°$ · UV $= \varepsilon_{235} = 14\,400$.
IR: 2250, 1770, 1680, 1640 $cm^{-1}$.

2. 5,0 g 3-(7α-Cyano-17β-hydroxy-15β,16β-methylen-3-oxo-4-androsten-17α-yl)-propionsäurelacton werden in 300 ml absolutem Tetrahydrofuran gelöst und auf —70°C abgekühlt. Dazu tropft man 35 ml 1,2molare Diisobutylaluminiumhydridlösung in Toluol und rührt 3 Stunden bei —70°C nach, zersetzt mit 200 ml gesättigter Zitronensäurelösung, extrahiert mit Dichlormethan, wäscht mit Wasser neutral, trocknet über Natriumsulfat und engt im Vakuum ein. Das erhaltene Rohprodukt wird durch Chromatographie an Kieselgel mit Dichlormethan/Aceton gereinigt. Man erhält 3,9 g 3β,5'-Dihydroxy-15β,16β-methylen-4-androsten-[17(β-1') spiro-2']-perhydrofuran-7α-carbonitril vom Schmelzpunkt 226—228°C.
IR: 3650, 3500, 2250, 1710 $cm^{-1}$.

3. 3,65 g 3β,5'-Dihydroxy-15β,16β-methylen-4-androsten [17(β-1')-spiro-2']-perhydrofuran-7α-carbonitril werden in 75 ml Dimethylformamid gelöst und mit 2,25 g Imidazol und 4,05 g tert.-Butyl-dimethyl-silylchlorid 3 Stunden bei Raumtemperatur gerührt, in 1 Liter Eiswasser gegeben, der ausgefallene Niederschlag abfiltriert, in Dichlormethan aufgenommen, über Natriumsulfat getrocknet und im Vakuum eingeengt. Das erhaltene Rohprodukt wird an Kieselgel mit Hexan/Essigester gereinigt. Man erhält 4,4 g 3,5'-Bis(tert.Butyldimethyl-silyloxy)-15β,16β-methylen-4-androsten-[(17β-1')-spiro-2']perhydrofuran-7α-carbonitril.
IR: 2250 $cm^{-1}$.

4. 4,3 g 3,5'-Bis(tert.-Butyl-dimethylsilyloxy)-15β,16β-methylen-4-androsten[(17β-1')-spiro-2']-perhydrofuran-7α-carbonitril werden in 100 ml absolutem Toluol gelöst und bei —70°C mit 13 ml 1,2molarer Diisobutylaluminiumhydridlösung in Toluol versetzt und drei Stunden bei dieser Temperatur gerührt. Anschließend zersetzt man mit 50 ml gesättigter Zitronensäurelösung, extrahiert die wäßrige Phase mit Ether, wäscht die vereinigten organischen Phasen mit Wasser neutral, trocknet über Natriumsulfat und engt im Vakuum ein. Man erhält 3,5 g 3,5'-Bis(tert.-Butyl-dimethylsilyloxy)-15β,16β-methylen-4-androsten[(17β-1')-spiro-2']-perhydrofuran-7α-carbaldehyd.
IR: 1725 $cm^{-1}$.

5. 1,1 g 3,5'-Bis(tert.-Butyl-dimethylsilyloxy)-15β,16β-methylen-4-androsten[(17β-1')-spiro-2']-perhydrofuran-7α-carbonitril werden in 50 ml Aceton gelöst und bei 0°C mit 2,7 ml Jones-Lösung versetzt und eine Stunde bei dieser Temperatur gerührt. Anschließend verdünnt man mit 300 ml Wasser und extrahiert mit Dichlormethan. Die vereinigten organischen Phasen werden mit 1n Natronlauge extrahiert, wäßrige Phase mit Dichlormethan gewaschen, mit kalter konzentrierter Schwefelsäure angesäuert, mit Dichlormethan extrahiert und mit Wasser neutral gewaschen, über Natriumsulfat getrocknet und im Vakuum eingeengt. Man erhält 528 g 3β-(7α-Carboxy-17β-hydroxy-15β,16β-methylen-3-oxo-4-androsten-17α-yl)-propionsäurelacton vom Schmelzpunkt 260—262°C.
UV: $\varepsilon_{238} = 16\,900$.

B) 3β-(7α-Carboxy-17β-hydroxy-6α-methyl-15β,16β-methylen-3-oxo-4-androsten-17α-yl)-propionsäurelacton:

1. 10 g 3-(17β-Hydroxy-15β,16β-methylen-3-oxo-4-androsten-17α-yl)-propionsäurelacton (Herstellung s. DE-OS 2 652 761) werden die Substanz zu einer Suspension von 19 ml Phosphoroxychlorid, 300 ml Chloroform, 10 g Natriumacetat und 300 ml Methylal gegeben und zum Sieden erhitzt. Dazu tropft man innerhalb von 2 Stunden 19 ml Phosphoroxychlorid und kocht für weitere 2 Stunden. Nach dem Abkühlen tropft man gesättigte Natriumcarbonatlösung zu, trennt die organische Phase ab und engt im Vakuum ein. Das erhaltene Rohprodukt wird durch Chromatographie an Kieselgel mit Dichlormethan/Aceton gereinigt. Man erhält 7,2 g 3-(17β-Hydroxy-6,6;15β,16β-bismethylen-3-oxo-4-androsten-17α-yl)-propionsäurelacton vom Schmelzpunkt 170,5—172,5.
UV: $\varepsilon_{261} = 11\,300$.

2. 7 g 3-(17β-Hydroxy-6,6;15β,16β-bis-methylen-3-oxo-4-androsten-17α-yl)-propionsäurelacton werden in 250 ml Ethanol mit 3,5 g wasserfreiem Natriumacetat und 500 mg Palladium auf Kohle 5%ig versetzt und in der Siedehitze über 8 Stunden 1,4 ml Cyclohexan in 40 ml Ethanol portionsweise zugegeben. Es wird dann vom Katalysator abfiltriert und das Filtrat im Vakuum eingeengt. Das erhaltene Rohprodukt wird durch Chromatographie an Kieselgel mit Dichlormethan/Aceton gereinigt. Man erhält 5,9 g 3-(17β-Hydroxy-6-methyl-15β,16β-methylen-3-oxo-4,6-androstadien-17α-yl)-propionsäurelacton vom Schmelzpunkt 228,5—231°C.
UV: $\varepsilon_{290} = 23\,800$.

3. 6,8 g 3-(17β-Hydroxy-6-methyl-15β,16β-methylen-3-oxo-4,6-androstadien-17α-yl)-propionsäurelacton werden in 150 ml absolutem Tetrahydrofuran mit 35 ml einer 2molaren Lösung von Diethylaluminiumcyanid in Toluol versetzt, 1 Stunde am Rückfluß gekocht, abgekühlt und in 100 ml 1n Natronlauge gegeben. Nach Extraktion mit Dichlormethan wäscht man mit 20%iger Schwefelsäu

re und Wasser, trocknet über Natriumsulfat und engt im Vakuum ein. Man erhält 6,7 g 3-(7α-Cyano-17β-hydroxy-6α-methyl-15β,16β-methylen-3-oxo-4-androsten-17α-yl)-propionsäurelacton als Rohrprodukt.

UV: $\varepsilon_{236}$ = 12 200.

4. 6,3 g 3-(7α-Cyano-17β-hydroxy-6α-methyl-15β,16β-methylen-3-oxo-4-androsten-17α-yl)-propionsäurelacton (Rohprodukt) werden in 400 ml absolutem Tetrahydrofuran gelöst und auf −70°C abgekühlt. Dazu tropft man 42 ml 1,2molare Diisobutylaluminiumhydridlösung in Toluol und rührt 3 Stunden bei −70°C nach, zersetzt mit 200 ml Zitronensäurelösung, extrahiert mit Dichlormethan, wäscht mit Wasser neutral, trocknet über Natriumsulfat und engt im Vakuum ein. Das erhaltene Rohprodukt wird durch Chromatographie an Kieselgel mit Dichlormethan/Aceton gereinigt. Man erhält 5,1 g 3β,5'-Dihydroxy-6α-methyl-15β,16β-methylen-4-androsten-[(17β-1')-spiro-2']perhydrofuran-7α-carbonitril.

IR: 3650, 3520, 2260, 1710 cm$^{-1}$.

5. 5 g 3β,5'-Dihydroxy-6α-methyl-15β,16β-methylen-4-androsten[(17β-1')-spiro-2]perhydrofuran-7α-carbonitril werden in 100 ml absolutem Dimethylformamid gelöst und mit 3 g Imidazol und 5,5 g tert.-Butyl-dimethylsilylchlorid 3 Stunden bei Raumtemperatur gerührt, in 1000 ml Eiswasser gegeben, der ausgefallene Niederschlag abfiltriert, in Dichlormethan aufgenommen, über Natriumsulfat getrocknet und im Vakuum eingeengt. Man erhält 6,2 g 3,5'-Bis(tert.-Butyl-dimethylsilyloxy)-6α-methyl-15β,16β-methylen-4-androsten[(17β-1')-spiro-2']-perhydrofuran-7α-carbonitril als Öl.

IR: 2260 cm$^{-1}$.

6. 6,0 g 3,5'-Bis(tert.-Butyl-dimethylsilyloxy)-6α-methyl-15β,16β-methylen-4-androsten[(17β-1')-spiro-2']-perhydrofuran-7α-carbonitril werden in 150 ml absolutem Toluol gelöst und bei −70°C mit 25 ml 1,2molarer Diisobutylaluminiumhydridlösung in Toluol versetzt eine Stunde bei −70°C und 2 Stunden bei −20°C gerührt. Anschließend zersetzt man mit 100 ml gesättigter Zitronensäurelösung, extrahiert die wäßrige Phase mit Ether, wäscht die vereinigten organischen Phasen mit Wasser neutral, trocknet über Natriumsulfat und engt im Vakuum ein. Nach Chromatographie an Kieselgel mit Hexan/Essigester erhält man 4,9 g 3,5'-Bis(tert.-Butyl-dimethylsilyloxy)-6α-methyl-15β,16β-methylen-4-androsten[(17β-1')-spiro-2']-perhydrofuran-7α-carbaldehyd als Öl.

IR: 1730 cm$^{-1}$.

7. 4,5 g 3,5'-Bis(tert.-Butyl-dimethylsilyloxy)-6α-methyl-15β,16β-methylen-4-androsten[(17β-1')-spiro-2']-perhydrofuran-7α-carbaldehyd werden in 125 ml Aceton gelöst und bei 0°C mit 10 ml Jones-Lösung versetzt und 2 Stunden bei dieser Temperatur gerührt. Anschließend verdünnt man mit 500 ml Wasser und extrahiert mit Dichlormethan. Die vereinigten organischen Phasen werden mit 1n Natronlauge extrahiert, die wäßrige Phase mit Dichlormethan gewaschen und mit kalter konzentrierter Schwefelsäure angesäuert, mit Dichlormethan extrahiert, mit Wasser neutralgewaschen, über Natriumsulfat getrocknet und im Vakuum eingeengt. Man erhält 2,45 g 3β-(7α-Carboxy-17β-hydroxy-6α-methyl-15β,16β-methylen-3-oxo-4-androsten-17α-yl)-propionsäurelacton.

UV: $\varepsilon_{239}$ = 14 400.

Die erfindungsgemäßen Verbindungen werden nach an sich bekannten Methoden der Galenik zur Herstellung von Arzneimitteln für die orale und parenterale Applikation verwendet.

Die Dosierung der erfindungsgemäßen Verbindung beträgt beim Menschen 10—200 mg/Tag.

Die nachfolgenden Beispiele sollen das erfindungsgemäße Verfahren erläutern.

Beispiel 1

308 mg 3β-(7α-Carboxy-17β-hydroxy-15β,16β-methylen-3-oxo-4-androsten-17α-yl)-propionsäurelacton werden in 10 ml Tetrahydrofuran gelöst und mit etherischer Diazomethanlösung bis zur bleibenden Gelbfärbung der Lösung versetzt. Das überschüssige Reagenz wird mit Eisessig zersetzt. Danach wird im Vakuum eingeengt. Das erhaltene Rohprodukt wird an Kieselgel mit Dichlormethan/Aceton chromatographiert. Man erhält 275 mg 3-(7α-Methoxycarbonyl-17β-hydroxy-15β,16β-methylen-3-oxo-4-androsten-17α-yl)-propionsäurelacton vom Schmelzpunkt 257,5—259°C.

UV: $\varepsilon_{243}$ = 16 400.

Beispiel 2

250 mg 3-(7α-Methoxycarbonyl-17β-hydroxy-15β,16β-methylen-3-oxo-4-androsten-17α-yl)-propionsäurelacton werden in 100 ml Dioxan gelöst und mit 250 mg Dichlordicyanochinon 4 Stunden am Rückfluß gekocht und Anschließend wird filtriert und im Vakuum eingeengt. Der Rückstand wird in Chloroform aufgenommen; die Lösung wird mit Wasser, 1n Natronlauge und Wasser gewaschen, über Natriumsulfat getrocknet und im Vakuum eingeengt. Das erhaltene Rohprodukt wird mit Dichlormethan/Aceton chromatographiert. Man erhält 172 mg 3-(7α-Methoxycarbonyl-17β-hydroxy-15β,16β-methylen-3-oxo-1,4-androstadien-17α-yl)-propionsäurelacton.
UV: $\varepsilon_{241} = 15\,900$.

## Beispiel 3

150 mg 3β-(7α-Carboxy-17β-hydroxy-15β,16β-methylen-3-oxo-4-androsten-17α-yl)-propionsäurelacton werden in 3 ml absolutem Tetrahydrofuran gelöst, mit 0,1 ml Triethylamin versetzt und auf 0°C abgekühlt. Dazu tropft man 0,05 ml Chlorameisensäurebutylester und rührt 1 Stunde bei dieser Temperatur, filtriert den ausgefallenen Niederschlag ab und engt im Vakuum ein. Der erhaltene Rückstand wird in Ethanol gelöst und 48 Stunden am Rückfluß gekocht. Nach dem Einengen im Vakuum reinigt man durch präparative Schichtchromatographie. Man erhält 109 mg 3-(7α-Ethoxycarbonyl-17β-hydroxy-15β,16β-methylen-3-oxo-4-androsten-17α-yl)-propionsäurelacton.
IR: 1770, 1725, 1675, 1620 $cm^{-1}$.

## Beispiel 4

150 mg 3β-(7α-Carboxy-17β-hydroxy-15β,16β-methylen-3-oxo-4-androsten-17α-yl)-propionsäurelacton werden in 3 ml absolutem Tetrahydrofuran gelöst, mit 0,1 ml Triethylamin versetzt und auf 0°C abgekühlt. Dazu tropft man 0,05 ml Chlorameisensäurebutylester und rührt 1 Stunde bei dieser Temperatur, filtriert den ausgefallenen Niederschlag ab und engt im Vakuum ein. Der erhaltene Rückstand wird in n-Propanol gelöst und 72 Stunden am Rückfluß gekocht. Nach dem Einengen im Vakuum reinigt man durch präparative Schichtchromatographie. Man erhält 87 mg 3-(7α-Propyloxycarbonyl-17β-hydroxy-15β,16β-methylen-3-oxo-4-androsten-17α-yl)-propionsäurelacton.

## Beispiel 5

205 mg 3-(7α-Methoxycarbonyl-17β-hydroxy-15β,16β-methylen-3-oxo-1,4-androstadien-17α-yl)-propionsäurelacton werden in Methanol suspendiert und mit 28 mg Kaliumhydroxid in 0,5 ml Wasser 16 Stunden bei Raumtemperatur und 1 Stunde bei 60°C gerührt. Dann wird im Vakuum eingeengt. Das erhaltene Öl wird in wenig Ethanol aufgenommen und mit Ether ausgefällt. Man erhält 123 mg 3-(7α-Methoxy-carbonyl-17β-hydroxy-15β,16β-methylen-3-oxo-1,4-androstadien-17α-yl)-propionsäurekaliumsalz.

## Beispiel 6

a) 825 mg 3-(7α-Methoxycarbonyl-17β-hydroxy-15β,16β-methylen-3-oxo-4-androsten-17α-yl)-propionsäurelacton werden in 20 ml absolutem Tetrahydrofuran gelöst und bei −70°C mit 1,8 ml 1,2molarer Diisobutylaluminiumhydrid-Lösung in Toluol versetzt und 2 Stunden bei dieser Temperatur belassen. Danach wird das überschüssige Reagenz mit 25 ml gesättigter Zitronensäurelösung zersetzt, das Reaktionsgemisch mit Dichlormethan extrahiert und die organische Phase mit Wasser neutralgewaschen, über Natriumsulfat getrocknet und im Vakuum eingeengt. Man erhält 738 mg 3β,5′-Dihydroxy-15β,16β-methylen-4-androsten[(17β-1′)-spiro-2′]-perhydrofuran-7α-carbonsäuremethylester, der roh weiterverarbeitet wird.
IR: 3580, 1730 $cm^{-1}$.

b) 730 mg 3β,5′-Dihydroxy-15β,16β-methylen-4-androsten[(17β-1′)-spiro-2′]-perhydrofuran-7α-carbonsäuremethylester werden in 10 ml iso-Propanol und 1 ml Wasser gelöst und mit 150 mg Natriumborhydrid 4 Stunden am Rückfluß gekocht. Danach wird mit 20 ml 0,1n Schwefelsäure versetzt und mit Dichlormethan extrahiert. Die organische Phase wird mit Wasser neutralgewaschen, über Natriumsulfat getrocknet und im Vakuum eingeengt. Das erhaltene Rohprodukt wird durch Säulenchromatographie an Kieselgel mit Dichlormethan/Aceton gereinigt. Man erhält 662 mg 3β-17β-Dihydroxy-15β,16β-methylen-17α-(3-hydroxypropyl)-4-androsten-7α-carbonsäuremethylester.
IR: 3600, 1730 $cm^{-1}$.

## Beispiel 7

1,2 g 3β-(7α-Carboxy-17β-hydroxy-6α-methyl-15β,16β-methylen-3-oxo-4-androsten-17α-yl)propionsäurelacton werden in 25 ml Tetrahydrofuran gelöst und mit etherischer Diazomethanlösung bis zur bleibenden Gelbfärbung versetzt. Das überschüssige Reagenz wird mit Eisessig zersetzt. Danach wird im Vakuum eingeengt. Das erhaltene Rohprodukt wird an Kieselgel mit Dichlormethan/Aceton chro-

matographiert. Man erhält 952 mg 3-(7α-Methoxycarbonyl-17β-hydroxy-6α-methyl-15β,16β-methylen-3-oxo-4-androsten-17α-yl)-propionsäurelacton.
UV: $\varepsilon_{241}$ = 17 500.

Beispiel 8

750 mg 3-(7α-Methoxycarbonyl-17β-hydroxy-6α-methyl-15β,16β-methylen-3-oxo-4-androsten-17α-yl)-propionsäurelacton werden in 20 ml absolutem Dioxan gelöst und mit 750 mg Dichlordicyanochinon 4 Stunden am Rückfluß gekocht. Anschließend wird filtriert und im Vakuum eingeengt. Das erhaltene Rohprodukt wird mit Dichlormethan/Aceton chromatographiert. Man erhält 552 mg 3-(7α-Methoxycarbonyl-17β-hydroxy-6α-methyl-15β,16β-methylen-3-oxo-4-androsten-17α-yl)-propionsäurelacton.
UV: $\varepsilon_{241}$ = 16 600.

Beispiel 9

a) 855 mg 3-(7α-Methoxycarbonyl-17β-hydroxy-6α-methyl-15β,16β-methylen-3-oxo-4-androsten-17α-yl)-propionsäurelacton werden in 20 ml absolutem Tetrahydrofuran gelöst und bei −70°C mit 1,8 ml 1,2molarer Diisobutylaluminiumhydrid-Lösung in Toluol versetzt und 2 Stunden bei dieser Temperatur belassen. Danach wird das überschüssige Reagenz mit 25 ml gesättigter Zitronensäurelösung zersetzt, das Reaktionsgemisch mit Dichlormethan extrahiert, und die organische Phase mit Wasser neutralgewaschen, über Natriumsulfat getrocknet und Vakuum eingeengt. Man erhält 725 mg 3β,5'-Dihydroxy-6α-methyl-15β,16β-methylen-4-androsten[17(β-1')-spiro-2']perhydrofuran-7α-carbonsäuremethylester, der roh weiterverarbeitet wurde.
IR: 3550, 1730 $cm^{-1}$.
b) 725 mg 3β,5'-Dihydroxy-6α-methyl-15β,16β-methylen-4-androsten[17(β-1')-spiro-2']perhydrofuran-7α-carbonsäuremethylester werden in 10 ml iso-Propanol und 1 ml Wasser gelöst und mit 150 mg Natriumborhydrid 4 Stunden am Rückfluß gekocht. Dann wird mit 20 ml 0,1n Schwefelsäure versetzt. Die organische Phase wird mit Dichlormethan extrahiert, mit Wasser neutralgewaschen, über Natriumsulfat getrocknet und im Vakuum eingeengt. Das erhaltene Rohprodukt wird durch Säulenchromatographie an Kieselgel gereinigt. Man erhält 642 mg 3β,17β-Dihydroxy-6α-methyl-15β,16β-methylen-17α(3-hydroxypropyl)-4-androsten-7α-carbonsäuremethylester.

Beispiel 10

Zu einer Lösung von 425 mg 3-(7α-Methoxycarbonyl-17β-hydroxy-15β,16β-methylen-3-oxo-4-androsten-17α-yl)-propionsäurelacton in 5 ml iso-Propanol gibt man 1 ml einer 1normalen methanolischen Kaliumhydroxidlösung und kocht 30 Minuten am Rückfluß. Nach dem Abkühlen gießt man in 50 ml eiskalten Ether, filtriert den Niederschlag ab und wäscht mit Ether nach. Nach dem Trocknen im Vakuum erhält man 378 mg 3-(7α-Methoxycarbonyl-17β-hydroxy-15β,16β-methylen-3-oxo-4-androsten-17α-yl)-propionsäure Kaliumsalz.

**Patentansprüche**

1. 7α-Alkoxycarbonyl-15β,16β-methylen-4-androsten-Verbindungen der allgemeinen Formel I

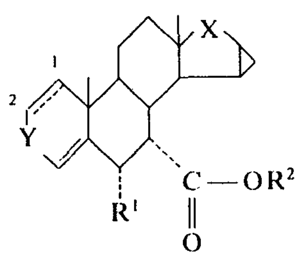

(I)

worin

$R^1$ ein Wasserstoffatom oder eine Methylgruppe,
$R^2$ eine Alkylgruppe mit 1 bis 4 Kohlenstoffatomen,
X die Gruppierung

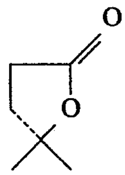

oder

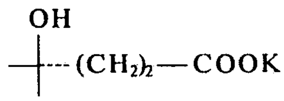

und
Y eine Carbonylgruppe

bedeuten und die Bindung zwischen den Kohlenstoffatomen 1 und 2 gesättigt ist oder eine Doppelbindung darstellt, oder

X die Gruppierung

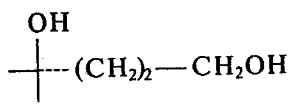

und
Y die Gruppe

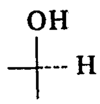

bedeuten, wenn die Bindung zwischen den Kohlenstoffatomen 1 und 2 eine Einfachbindung darstellt.

2. 3-(7α-Methoxycarbonyl-17β-hydroxy-15β,16β-methylen-3-oxo-4-androsten-17α-yl)propionsäurelacton.

3. 3-(7α-Methoxycarbonyl-17β-hydroxy-15β,16β-methylen-3-oxo-1,4-androstadien-17α-yl)propionsäurelacton.

4. Kaliumsalz der 3-(7α-Methoxycarbonyl-17β-hydroxy-15β,16β-methylen-3-oxo-1,4-androstadien-17α-yl)propionsäure.

5. 3-(7α-Methoxycarbonyl-17β-hydroxy-6α-methyl-15β,16β-methylen-3-oxo-4-androsten-17α-yl)propionsäurelacton.

6. 3-(7α-Methoxycarbonyl-17β-hydroxy-6α-methyl-15β,16β-methylen-3-oxo-1,4-androstadien-17α-yl)propionsäurelacton.

7. 3-(7α-Methoxycarbonyl-17β-hydroxy-15β,16β-methylen-3-oxo-4-androsten-17α-yl)propionsäure Kaliumsalz.

8. Verfahren zur Herstellung von 7α-Alkoxycarbonyl-15β,16β-methylen-4-androsten-Verbindung der allgemeinen Formel I

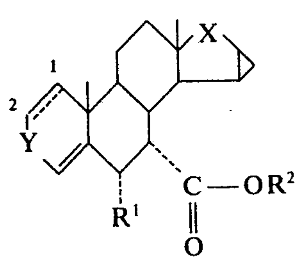

(I)

worin

$R^1$ ein Wasserstoffatom oder eine Methylgruppe,
$R^2$ eine Alkylgruppe mit 1 bis 4 Kohlenstoffatomen,
X die Gruppierung

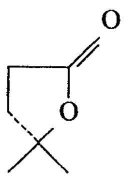 oder 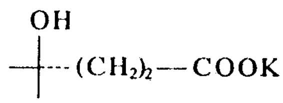

und
Y eine Carbonylgruppe

bedeuten und die Bindung zwischen den Kohlenstoffatomen 1 und 2 gesättigt ist oder eine Doppelbindung darstellt, oder

X die Gruppierung

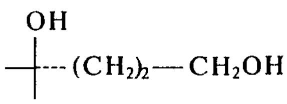

und
Y die Gruppe

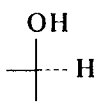

bedeuten, wenn die Bindung zwischen den Kohlenstoffatomen 1 und 2 eine Einfachbindung darstellt, dadurch gekennzeichnet, daß man in an sich bekannter Weise eine Verbindung der allgemeinen Formel II

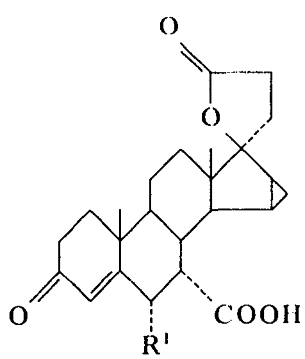

(II)

worin $R^1$ die obengenannte Bedeutung besitzt, zu einem nieder-Alkyl-Ester umsetzt und gegebenenfalls die $\Delta^1$-Doppelbindung einführt und/oder den Lacton-Ring zum entsprechenden Propionsäure-Kalium-Salz öffnet oder zu den entsprechenden 3$\beta$,17$\beta$-Dihydroxy-15$\beta$,16$\beta$-methylen-17$\alpha$-(3-hydroxypropyl)-4-androsten-7$\alpha$-alkoxycarbonylverbindungen reduziert.

9. Arzneimittel auf Basis der Verbindungen gemäß den Ansprüchen 1 bis 7.

**Claims**

1. 7$\alpha$-Alkoxycarbonyl-15$\beta$,16$\beta$-methylene-4-androstene compounds of general formula I

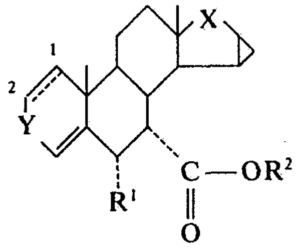

(I)

wherein

$R^1$ is hydrogen or methyl,
$R^2$ is alkyl of 1 to 4 carbon atoms,
X is the grouping

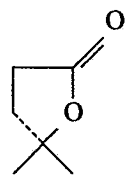

or

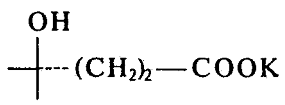

and
Y is carbonyl, and the bond between carbons 1 and 2 is saturated or is a double bond, or
X is the grouping

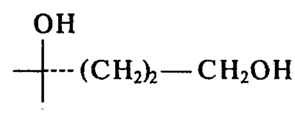

and
Y is

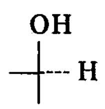

when the bond between carbons 1 and 2 is a single bond.

2. 3-(7$\alpha$-Methoxycarbonyl-17$\beta$-hydroxy-15$\beta$,16$\beta$-methylene-3-oxo-4-androsten-17$\alpha$-yl)propiolactone.

3. 3-(7$\alpha$-Methoxycarbonyl-17$\beta$-hydroxy-15$\beta$,16$\beta$-methylene-3-oxo-1,4-androstadien-17$\alpha$-yl)propiolactone.

4. Potassium 3-(7$\alpha$-methoxycarbonyl-17$\beta$-hydroxy-15$\beta$,16$\beta$-methylene-3-oxo-1,4-androstadien-17$\alpha$-yl)propionate.

5. 3-(7$\alpha$-Methoxycarbonyl-17$\beta$-hydroxy-6$\alpha$-methyl-15$\beta$,16$\beta$-methylene-3-oxo-4-androsten-17$\alpha$-yl)propiolactone.

6. 3-(7$\alpha$-Methoxycarbonyl-17$\beta$-hydroxy-6$\alpha$-methyl-15$\beta$,16$\beta$-methylene-3-oxo-1,4-androstadien-17$\alpha$-yl)propiolactone.

7. Potassium 3-(7$\alpha$-methoxycarbonyl-17$\beta$-hydroxy-15$\beta$,16$\beta$-methylene-3-oxo-4-androsten-17$\alpha$-yl)propionate.

8. Process for the preparation of 7$\alpha$-alkoxycarbonyl-15$\beta$,16$\beta$-methylene-4-androstene compounds of general formula I

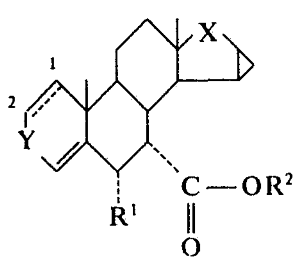

(I)

wherein

$R^1$ is hydrogen or methyl,
$R^2$ is alkyl of 1 to 4 carbon atoms,
X is the grouping

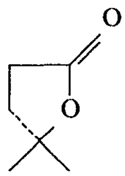

or

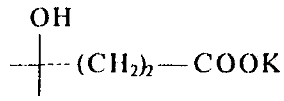

and
Y is carbonyl, and the bond between carbons 1 and 2 is saturated or is a double bond, or
X is the grouping

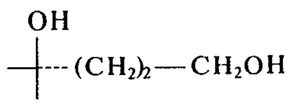

and
Y is

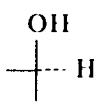

when the bond between carbons 1 and 2 is a single bond, characterised in that a compound of general formula II

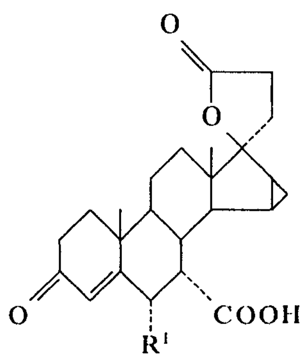

(II)

wherein $R^1$ has the meaning given above, is converted to a lower alkyl ester, in a known manner, and optionally the $\Delta^1$-double bond is introduced and/or the lactone ring is opened to give the corresponding potassium propionate salt or is reduced to give the corresponding 3β,17β-dihydroxy-15β,16β-methylene-17α-(3-hydroxypropy)-4-androsten-7α-alkoxycarbonyl compound.

9. Pharmaceutical compositions based on compounds according to claims 1 to 7.

**Revendications**

1. Alcoxycarbonyl-7α méthylène-15β,16β androstènes-4 répondant à la formule générale I:

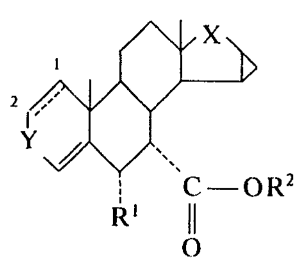

(I)

dans laquelle

$R^1$ représente un atome d'hydrogène ou un radical méthyle,
$R^2$ représente un radical alkyle contenant de 1 à 4 atomes de carbone,
X représente l'un des groupements:

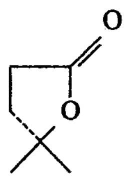 et 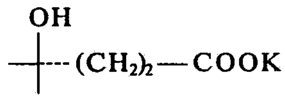

et
Y représente un radical carbonyle, auquel cas la liaison entre les atomes de carbone 1 et 2 est simple ou double, ou
X représente le groupement

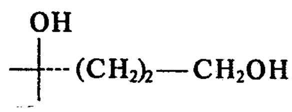

et
Y représente le radical

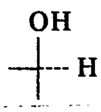

auquel cas la liaison entre les atomes de carbone 1 et 2 une liaison simple.

2. Lactone de l'acide (méthoxycarbonyl-7α hydroxy-17β méthylène-15β,16β oxo-3 androstène-4 yl-17α)-3 propionique.

3. Lactone de l'acide (méthoxycarbonyl-7α hydroxy-17β méthylène-15β,16β oxo-3 androstadiène-1,4-yl-17α)-3 propionique.

4. Sel potassique de l'acide (méthoxycarbonyl-7α hydroxy-17β méthylène-15β,16β oxo-3 androstadiène-1,4 yl-17α)-3 propionique.

5. Lactone de l'acide (méthoxycarbonyl-7α hydroxy-17β méthyl-6α méthylène-15β,16β oxo-3 androstène-4 yl-17α)-3 propionique.

6. Lactone de l'acide (méthoxycarbonyl-7α hydroxy-17β méthyl-6α méthylène-15β,16β oxo-3 androstadiène-1,4 yl-17α)-3 propionique.

7. Sel potassique de l'acide (méthoxycarbonyl-7α hydroxy-17β méthylène-15β,16β oxo-3 androstène-4 yl-17α)-3 propionique.

8. Procédé de préparation d'alcoxycarbonyl-7α méthylène-15β,16β androstènes-4 répondant à la formule générale I:

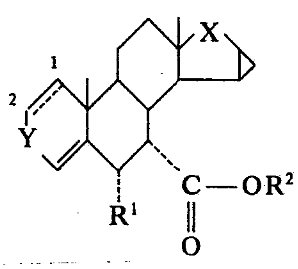

(I)

dans laquelle

$R^1$ représente un atome d'hydrogène ou un radical méthyle,
$R^2$ représente un radical alkyle contenant de 1 à 4 atomes de carbone,
X représente l'un des groupements:

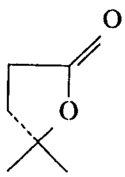 et 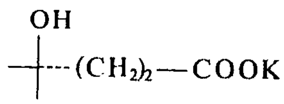

et
Y représente un radical carbonyle, auquel cas la liaison entre les atomes de carbone 1 et 2 est simple ou double, ou
X représente le groupement

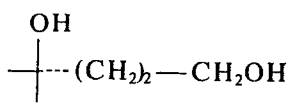

et
Y représente le radical

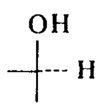

auquel cas la liaison entre les atomes de carbone 1 et 2 est une liaison simple,

procédé caractérisé en ce que, en opérant de manière connue, on fait réagir un composé répondant à la formule générale II:

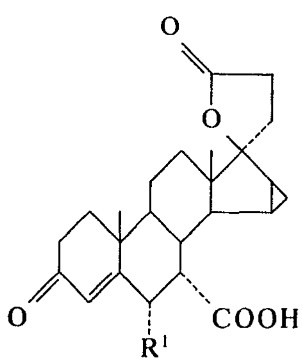

(II)

dans laquelle $R^1$ a la signification précédemment donnée, de manière à le convertir en un ester alkylique inférieur, et éventuellement on introduit la double liaison $\Delta^1$ et/ou on ouvre le noyau lactonique de manière à obtenir le propionate de potassium correspondat ou on effectue une réduction pour obtenir le dihydroxy-3β,17β alcoxycarbonyl-7α méthylène-15β,16β (hydroxy-3 propyl)-17α androstène-4 correspondant.

9. Médicaments à base de composés selon l'une quelconque des revendications 1 à 7.